# EUROPEAN PATENT APPLICATION

(11) **EP 4 285 910 A1**
(43) Date of publication of application: **06.12.2023**
(21) Application number: 21928214.2
(22) Date of filing: 18.10.2021
(51) Int. Cl.: A61K 31/7034, A61P 25/00

(54) **PHARMACEUTICAL COMPOSITION FOR TREATMENT OF NEUROINFLAMMATION, COMPRISING ELEUTHEROSIDE B AS ACTIVE INGREDIENT**

(30) Priority: 26.02.2021 KR 20210026545
(71) Applicant: Yep Bio Co. Ltd., Anyang-si, Gyeonggi-do 14056 (KR)
(72) Inventor: PARK, Chi Hu, Hwaseong-si Gyeonggi-do 18237 (KR); KIM, Hyoung Shik, Hwaseong-si Gyeonggi-do 18237 (KR); CHOI, Yoori, Seoul 07579 (KR)
(74) Representative: Roos, Peter
(86) International application number: PCT/KR2021/014493
(87) International publication number: WO 2022/181920

(57) **Abstract**

The present invention relates to a pharmaceutical composition for the treatment of neuroinflammation comprising eleutheroside B as an active ingredient, and more particularly, to a pharmaceutical composition for treating neuroinflammatory disease in patients with the increased activity of astrocytes or microglia, comprising eleutheroside B or a pharmaceutically acceptable salt thereof as an active ingredient. That is, it is possible to alleviate inflammation by effectively suppressing the activity of active microglia and astrocytes, increasing the secretion of anti-inflammatory cytokines, and to protect the damage of activated microglia and astrocytes to brain nerve cells.

## Description

### [Technical Field]

The present invention relates to a pharmaceutical composition for the treatment of neuroinflammation comprising eleutheroside B as an active ingredient, and more particularly, to a pharmaceutical composition for treating neuroinflammatory disease in patients with the increased activity of astrocytes or microglia, comprising eleutheroside B or a pharmaceutically acceptable salt thereof as an active ingredient.

### [Background Art]

Neurodegenerative diseases are diseases in which mental functions are degenerated due to the progressive structural and functional loss of neurons. Degenerative neurological diseases are accompanied by symptoms such as dementia, extrapyramidal tract abnormalities, cerebellar abnormalities, sensory disorders, and motor disorders by the progression of nerve cell degeneration in specific parts of the nervous system, and abnormalities appear in several areas at the same time and thus symptoms may occur in combination. Therefore, diseases are diagnosed according to the clinical features of patients, but symptoms appear in a variety of ways, and many different diseases often show common clinical symptoms, which makes it difficult to diagnose. Symptoms of these degenerative neurological diseases occur slowly and often develop with aging. Once developed, the disease progresses continuously over several years or decades until death, and it is difficult to fundamentally treat it, so the social burden is very large. Genetic factors according to family history may act as a cause of onset, but epigenetic factors are also known to play an important role. Neurodegenerative diseases are largely classified according to their clinical symptoms, i.e., progressive dementia (Alzheimer's disease, etc.), neurological abnormalities (Pick's disease, etc.), postural and motor abnormalities (Parkinson's disease, etc.), progressive ataxia, muscular atrophy and weakness, sensory and motor disorders, etc. Among them, Alzheimer's disease, which is the most prevalent neurodegenerative disease with the highest prevalence at 6.54% among those aged 65 or older, accounts for 71.3% of all dementia, and β-amyloid plaque and neuroinflammation and cytotoxicity due to neurofibrillary tangles are attracting attention as a direct cause.

In the Alzheimer's disease, the secretion of the neurotransmitter acetylcholine is reduced, the interaction between nerve cells is blunted, the hippocampus, forebrain, and temporal lobe are mainly damaged, and as the disease progresses, about 30 to 40% of the nerve cells in this part is lost. As a result of the study, it was found that Alzheimer's disease is pathologically characterized by brain atrophy in the cerebral cortex or hippocampus, senile plaques and neuroinflammation in the brain from the onset, resulting in the deposition of neurofibrillary tangles. In addition, observing the pathogenesis of Alzheimer's disease, amyloid precursor proteins (APP) are abnormally decomposed 15 to 20 years before the onset of the disease, and β-amyloid protein accumulates, and at the same time, neuroinflammation occurs due to the activation of astrocytes and microglia, and then, as the disease progresses, neurofibrillary tangles accumulate due to hyperphosphorylation of tau. According to the results of studies so far, reports have been published that amyloid alone does not induce neuronal death, but when inflammation is accompanied, abnormal tau protein propagation is induced, and when inflammation progresses with amyloid, and once tau protein begins to be deposited, cognitive function does not recover even if tau is removed. As described above, the importance of neuroinflammation was emphasized as a cause of Alzheimer's in the early stage of the study, along with the formation of amyloid plaques and nerve fiber bundles. Due to the single cell analysis method developed in 2017, studies on the diversity of inflammation and disease conditions according to brain regions began.

It has been reported that neuroinflammation occurs due to the activation of microglia and astrocytes among glial cells in the brain, which leads to the death of neurons and occurs from the early stage of deposition of amyloid. Among them, microglia are immune cells residing in the central nervous system (CNS) and are known to be activated by external stimuli to induce immune and inflammatory responses. In particular, when microglia are activated with substances such as bacterial endotoxin lipopolysaccharide (LPS), interferon-γ, beta-amyloid or ganglioside, unlike microglia in a normal state, phagocytosis is active, cell proliferation is active, pro-inflammatory cytokines TNF-α, IL-1β and IL-6, chemokines, iNOS (inducible nitric oxide synthase), COX-2 (cyclooxygenase-2), and other genes are expressed to produce inflammatory mediators. Among them, the activation of microglia by beta-amyloid, the causative agent of Alzheimer's disease, has a net effect of removing damaged cells and protecting nerve cells from invading bacteria or viruses, but by activating astrocytes and nitric oxide (NO) produced by the activity of astrocytes and iNOS, and prostaglandins produced by COX-2, TNF-α, etc., are toxic to nerve cells and result in the death of nerve cells, and as a result, the activation of microglial cells causes neuroinflammation, which in turn aggravates Alzheimer's disease.

On the other hand, astrocytes play an important role in maintaining neurotransmission and brain homeostasis activities as well as brain development, removing substances through the BBB and supplying nutrients to nerve cells. Astrocytes in the brain have been found to play a role in assisting nerve cell activity by properly removing neurotransmitters secreted by nerve cells through the BBB or by regulating ion concentrations in the brain. In addition, it has been found that neural stem cells play a role in differentiating into neurons. However, astrocytes are actively proliferated and swelling occurs when the brain is injured, and it is activated into active astrocytes, such as astrogliosis. These active astrocytes have been found to activate microglia, induce synaptic function degradation and secretion of pro-inflammatory cytokines, and lead to neuronal death. It has been reported that such active astrocytes are observed in AIDS dementia, brain damage, ischemic brain disease, Alzheimer's disease, etc., and such continuous activation of astrocytes causes neuroinflammation and eventually leads to the death of neurons.

Therefore, the control of neuroinflammation due to the activation of astrocytes and microglia, which starts from the early stage of abnormal protein deposition in degenerative neurological diseases such as Alzheimer's disease, is very important even before cognitive function is damaged, and the development of therapeutic agents that control this is a very important factor in preventing nerve damage and memory degeneration, so research on this is necessary.

### [Disclosure]

### [Technical Problem]

The present invention provides a pharmaceutical composition for treating neuroinflammatory disease in patients with increased activity of astrocytes and microglia, comprising eleutheroside B or a pharmaceutically acceptable salt thereof as an active ingredient.

### [Technical Solution]

The present invention provides a pharmaceutical composition for treating neuroinflammatory disease in patients with increased activity of astrocytes and microglia, comprising eleutheroside B or a pharmaceutically acceptable salt thereof as an active ingredient.

The composition inhibits the activity of astrocytes or microglia to maintain the cell area small, increases the secretion of anti-inflammatory cytokines and inhibits the secretion of pro-inflammatory cytokines, and thus it can inhibit the induction of inflammation in neuronal cells.

In addition, the composition may have an effect of preventing or treating neurodegenerative diseases by suppressing the neuroinflammation, and the neurodegenerative diseases can be selected from the group consisting of Alzheimer's disease, Parkinson's disease, multiple sclerosis, cerebral infarction, stroke, epilepsy, multiple nerve atrophy, and Huntington's disease.

In addition, the composition can promote differentiation of brain neurons by activating neurites in neural stem cells.

### [Advantageous Effects]

The present invention relates to a pharmaceutical composition for the treatment of neuroinflammation comprising eleutheroside B as an active ingredient, and more particularly, to a pharmaceutical composition for treating neuroinflammatory disease in patients with the increased activity of astrocytes or microglia, comprising eleutheroside B or a pharmaceutically acceptable salt thereof as an active ingredient. That is, it is possible to alleviate the inflammation by effectively suppressing the activity of active microglia and astrocytes, increasing the secretion of anti-inflammatory cytokines and inhibiting the secretion of pro-inflammatory cytokines, and to protect the damage of activated microglia and astrocytes to brain nerve cells.

In addition, as described above, the pharmaceutical composition comprising eleutheroside B according to the present invention has the effect of preventing or treating neurodegenerative diseases by suppressing the neuroinflammation, activating neurite in neural stem cells, and promoting the differentiation of neurons in cranial neural stem cells.

### [Description of Drawings]

FIG. 1 is a graph showing the results of confirming the survival rate in microglia for confirming the toxicity according to the increase in the concentration of eleutheroside B.
FIG. 2 shows the process and method for verifying the neuroinflammation inhibitory effect using astrocytes.
FIG. 3 shows an immunocytochemistry result of measuring GFAP (green) fluorescence intensity before and after drug treatment after inducing neuroinflammation in astrocytes with LPS.
FIG. 4 is graphs showing quantification and comparison of effects on cell number, area, and GFAP expression level when LPS-treated astrocytes were treated with eleutheroside B and donepezil (an FDA-approved drug).
FIG. 5 is graphs showing the comparison of the effects on cytokine secretion when LPS-treated astrocytes were treated with eleutheroside B and donepezil.
FIG. 6 shows the experimental outline for observation of changes in neuroinflammation of microglial cells by drug treatment.
FIG. 7 shows the measurement of lba1 (green) fluorescence intensity before and after drug treatment after induction of the neuroinflammation in microglial cells with LPS.
FIG. 8 is graphs showing the comparison of the effects on cell number and Iba1 (green) expression level when LPS-treated microglia were treated with eleutheroside B and donepezil.
FIG. 9 is a graph showing the comparison of the effects on the secretion of cytokines when LPS-treated microglia were treated with eleutheroside B and donepezil.
FIG. 10 shows the result of confirming the activity of neurites from neural stem cells according to the eleutheroside B treatment.
FIG. 11 is graphs showing the results of neuronal differentiation marker analysis (Sox2, Nestin, NeuroD, DCX, Tuj-1, NeuN) after treatment with eleutheroside B in Neuroblast cell lines.
FIG. 12 is a graph showing the changes in cognitive function in an Alzheimer's disease animal model administered with eleutheroside B.
FIG. 13 is brain immunohistochemistry results of the hippocampus in which active astrocytes in an animal model of Alzheimer's disease were changed by the administration of eleutheroside B. Green: GFAP <astroglia>, Blue: DAPI, Red: β-amyloid.
FIG. 14 is a graph showing the quantification of the degree of change in the activity of astrocytes by the administration of eleutheroside B in the brain immunohistochemistry of FIG. 13.
FIG. 15 is a graph showing the quantification of the degree of change in the activity of astrocytes in the CA1 region of the hippocampus by the administration of eleutheroside B in the brain immunohistochemistry of FIG. 13.
FIG. 16 shows brain immunohistochemistry results of the hippocampus in which the activity of microglial cells in an animal model of Alzheimer's disease was changed by the administration of eleutheroside B. Green: Iba1 <astrocytic cells>, Blue: DAPI, Red: β-amyloid.
FIG. 17 is a graph quantifying changes in microglial cells according to the treatment of eleutheroside B in the brain immunohistochemistry of FIG. 16.
FIG. 18 is a graph quantifying changes in microglia in the CA1 region of the hippocampus according to the treatment of eleutheroside B in the brain immunohistochemistry of FIG. 16.
FIG. 19 shows the result of confirming the passage of the BBB (blood brain barrier) according to the treatment of eleutheroside.

### [Best Mode]

Since the present invention can apply various changes and have various embodiments, specific embodiments will be illustrated in the drawings and described in detail. However, it should be understood that this is not intended to limit the present invention to specific embodiments, and includes all transformations, equivalents or substitutes included in the spirit and scope of the present invention. In describing the present invention, if it is determined that a detailed description of related known technologies may obscure the gist of the present invention, the detailed description will be omitted.

The present invention provides a pharmaceutical composition for treating neuroinflammatory disease in patients with the increased activity of astrocytes or microglia, comprising eleutheroside B or a pharmaceutically acceptable salt thereof as an active ingredient.

Conventional therapeutic agents for neurodegenerative diseases target the reduction of the secretion of acetylcholine, a neurotransmitter in the hippocampus, and are single-targeted types that promote or inhibit degradation, and most of them were limited to the purpose of relieving symptoms. Since these are chemically synthesized therapeutics, there is a problem in that they require a long period of research and high cost, and have side effects such as headache, anxiety, or gastrointestinal disorder. In addition, until recently, new treatments for the removal of amyloid plaques, neuroinflammation, and neurofibrillary tangles have been actively studied through mechanism studies on the causes of degenerative neurological diseases, but there are no new drugs that improve cognition and alleviate symptoms after 2014.

Since the excessive activation of microglia and astrocytes, which cause neuroinflammation, damages the nerves and deteriorates the memory, the present inventors have experimentally confirmed a method of suppressing neuroinflammation using eleutheroside B and completed the invention.

Hereinafter, a pharmaceutical composition for treating neuroinflammatory diseases according to specific embodiments of the present invention will be described in more detail.

Eleutheroside B is contained in the bark or leaves of Siberian ginseng, lilac, and Japanese privet, and has a melting point of 192°C as white needle-shaped crystals. It is known that eleutheroside B increases the weight of seminal vesicle and prostate, prevents atrophy of the seminal vesicle and the prostate of castrated animals, enhances stamina, prevents aging, and helps improve eyesight and hearing. The anti-fatigue and excitatory action of eleutheroside B is similar to that of ginseng saponin, and in particular, it is known to be effective against liver damage, and is reported to be effective for vasodilation, fatigue recovery, anticonvulsant, and antistress action.

A composition comprising eleutheroside B or a pharmaceutically acceptable salt thereof as an active ingredient according to an embodiment of the present invention has an effect of suppressing the neuroinflammation.

Microglia, which act as macrophages in the brain, are important cells that regulate the immune response in the central nervous system. Their activation plays an important role in maintaining CNS homeostasis by removing foreign substances caused by drugs or toxins and secreting nerve growth factors. However, when exposed to harmful stresses such as signals generated from damaged neurons, accumulation of abnormal proteins modified by external stimuli, and infiltration of pathogens, the activity of microglia is excessively increased, degenerative neurological diseases can be caused by excessively increasing the activity of microglial cells and damaging nerve cells. Therefore, a method for suppressing excessive activity of microglia can be a treatment method for neurodegenerative diseases.

That is, excessively activated microglia, unlike microglia in a normal state, activate phagocytosis, proliferate, and express pro-inflammatory cytokines and genes related to inflammation to produce inflammatory mediators.

Activation of microglia exhibits a positive effect of removing damaged cells and protecting nerve cells from bacteria or viruses invading from the outside, but has a harmful effect which activation of astrocytes, production of nitric oxide (NO), activation of inflammatory signals by COX-2, increased cytokines of TNF-α cause toxicity to nerve cells and the death of nerve cells. As a result, activation of microglia aggravates the damage to nerve cells and causes neurodegenerative diseases.

In addition, astrocytes are also known to play an important role in maintaining normal brain activity. In particular, they are known to play a role in synapse formation of nerve cells, regulation of synapse number, synaptic function, and differentiation of neural stem cells into neurons. However, if these astrocytes are excessively reactive, that is, if they are maintained in an excessively activated state, they activate microglial cells, cause the death of neurons, and induce the death of neighboring neurons, leading to degenerative neurological diseases. Therefore, inhibition of activation of active astrocytes may also be a new treatment method for neurodegenerative diseases.

The pharmaceutical composition according to an embodiment of the present invention can inhibit the activity of astrocytes or microglia, specifically, reactive astrocytes or active microglia can inhibit inflammation in brain nerve cells.

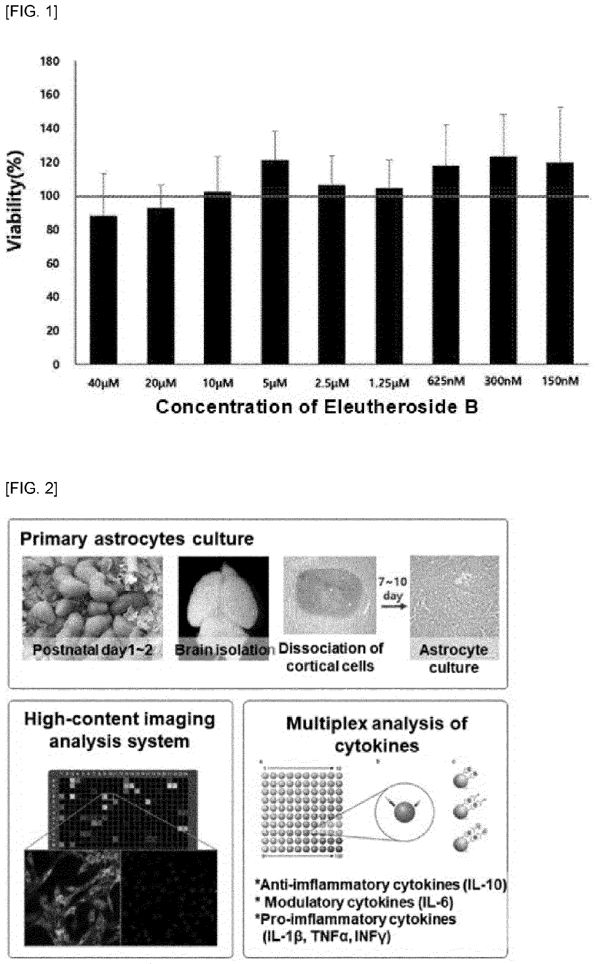

Example 4 of the present invention shows that eleutheroside B is safe as it has no cytotoxicity to microglia. As a result of confirming the survival rate according to the treatment with eleutheroside B, the survival rate was 92±13.3% at 20 uM and 88±24.8% at 40 uM (mean±standard deviation), and the survival rate tended to decrease. Therefore, it was confirmed that the stability was more than 100 times the effective concentration (FIG. 1).

Example 5 of the present invention shows the effect of inhibiting astrocyte activity according to eleutheroside B treatment *in-vitro.*

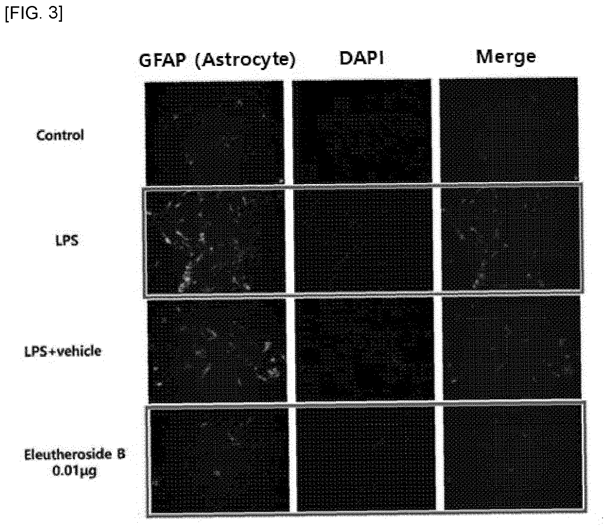

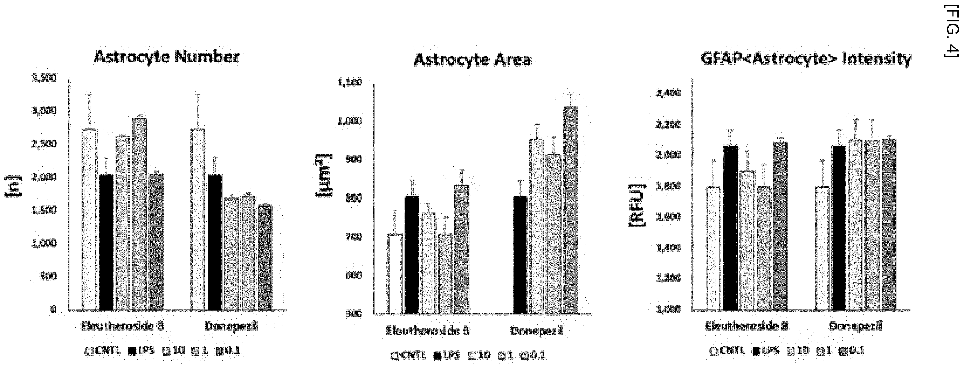

As shown in FIG. 3, after inducing activation of astrocytes with LPS and treating them with 0.01 µg of eleutheroside B, it was confirmed that the activity was reduced to almost the same intensity as that of the control, indicating that inflammation was suppressed. In addition, as shown in FIG. 4, treatment after activation of astrocytes by LPS at 1-10 nM drug concentration of eleutheroside B showed cell proliferation compared to LPS activity, and cell area and intensity of GFAP has been reduced. This is thought to reduce inflammation and inhibit apoptosis of astrocytes as the activity of astrocytes decreases. On the other hand, in the case of 1-10 nM drug concentration of donepezil, which is currently used as an Alzheimer's disease drug, about 10% of cell death was observed compared to LPS activity, the cell area was increased, and the intensity of GFAP was almost equal or slight increases were observed. This is thought to be due to the fact that donepezil cannot inhibit the activity of astrocytes, resulting in inflammation and consequent neuronal cell death.

As a result, it indicates that inflammation induced by the activity of astrocytes is not regulated by donepezil, which is currently used as a drug for Alzheimer's disease, but eleutheroside B can alleviate inflammation by inhibiting the activity of astrocytes.

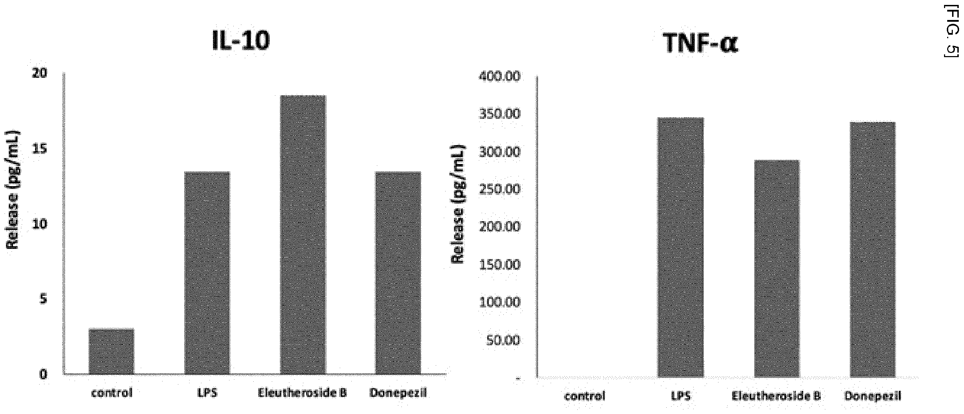

In addition, as shown in the multiplexing result of cytokine analysis in FIG. 5, an increase in cytokines was observed when LPS was treated in the control, and when eleutheroside B was treated, the anti-inflammatory cytokine IL-10 increased, but the secretion of pro-inflammatory cytokine TNF-α decreased, so that inflammation was regulated, and in contrast, donepezil did not affect cytokine secretion.

In addition, Example 6 of the present invention shows the effect of inhibiting microglia activity by *in-vitro* treatment with eleutheroside B.

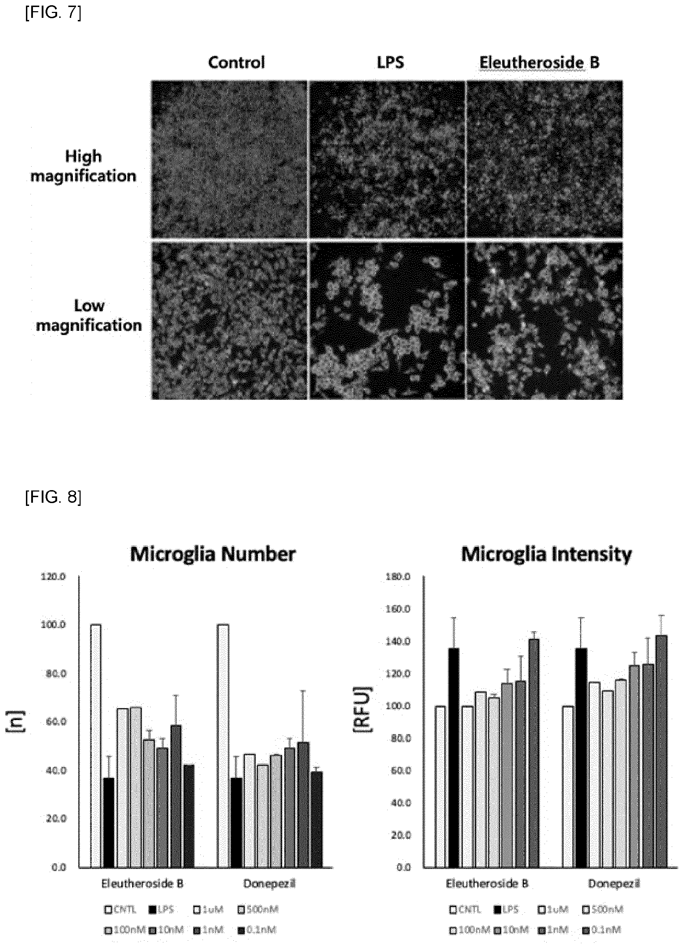

As shown in FIG. 7, after inducing activation of microglial cells with LPS, and then treatment with 10 nM of eleutheroside B, it was confirmed that the activity decreased with almost the same fluorescence intensity as the control.

As shown in FIG. 8, as a result of treating microglia with LPS, the number of cells decreased and the fluorescence intensity of Iba1 increased, showing an activation pattern of microglia. However, after treatment with eleutheroside B, the number of cells for which microglial apoptosis was inhibited increased, and a decrease in fluorescence intensity of Iba1 was observed, indicating that the activity was inhibited. Donepezil also showed a pattern of decreasing the fluorescence intensity of Iba1 and inhibiting microglia apoptosis, but showed a relatively less microglial activity inhibition pattern than eleutheroside B. This is a strong evidence that eleutheroside B can regulate microglia activity, and it was found that inflammation can be alleviated through the regulation of microglia activity by eleutheroside B.

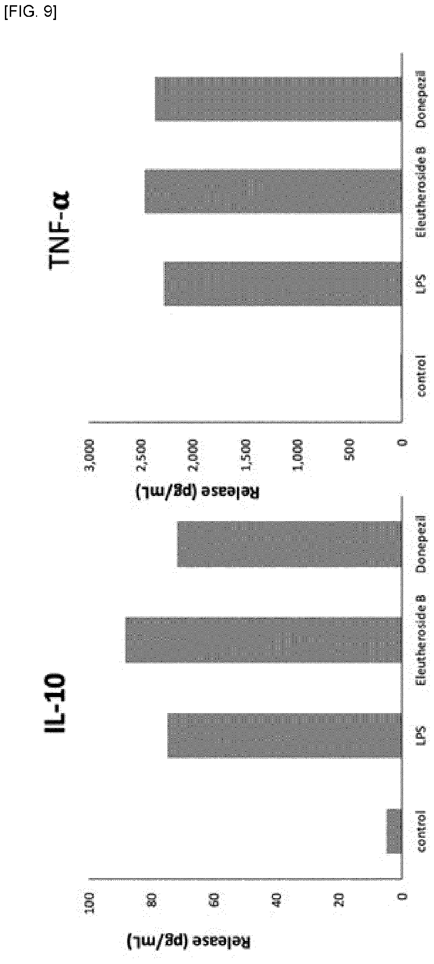

In addition, as shown in FIG. 9, as a result of cytokine analysis multiplexing using conditioned media after treatment of microglia with LPS and eleutheroside B, an increase in cytokines was observed after LPS treatment, as with the results in astrocytes. When eleutheroside B was treated, the anti-inflammatory cytokine IL-10 increased to control inflammation. In contrast, it was found that donepezil did not affect cytokine secretion.

Therefore, the pharmaceutical composition according to the present invention can control inflammation by inhibiting the activity of astrocytes or microglia, increasing the secretion of anti-inflammatory cytokines, and inhibiting the secretion of pro-inflammatory cytokines.

In addition, the pharmaceutical composition according to the present invention can have an effect of preventing or treating neurodegenerative diseases by inhibiting neuroinflammation, and the neurodegenerative diseases can be selected from the group consisting of Alzheimer's disease, Parkinson's disease, multiple sclerosis, cerebral infarction, stroke, epilepsy, multiple nerve atrophy, and Huntington's disease, but they are not limited thereto.

Furthermore, the composition can activate neurites in nerve cells and promote the nerve cell differentiation in neural stem cells.

In Example 7 of the present invention, the group treated with eleutheroside B was confirmed to have neurite activity in neurons differentiated from neural stem cells obtained from Huntington's disease transgenic mice compared to the untreated group.

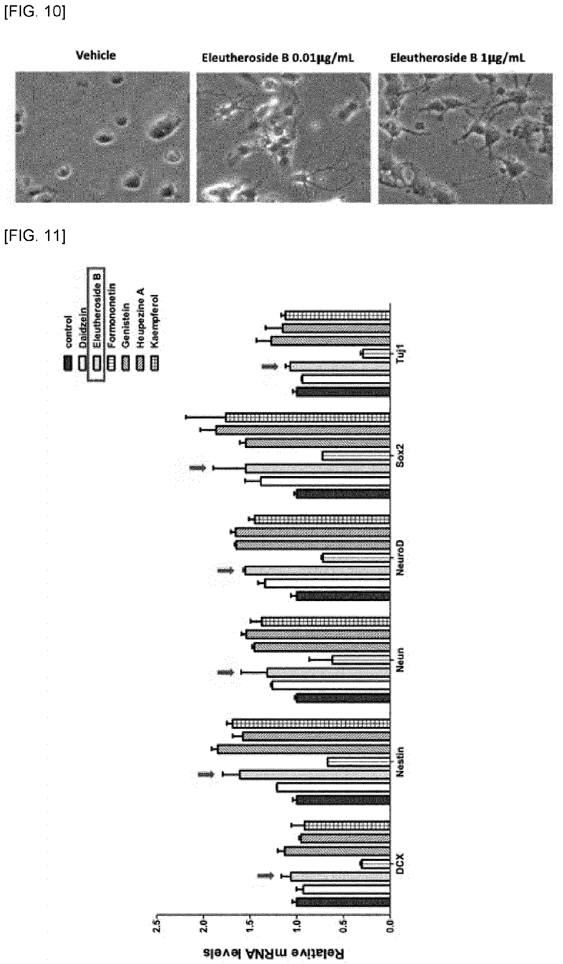

As shown in FIG. 10, compared to the control group in which neurites, which are characteristic of neurodegenerative diseases, were hardly seen, the groups treated with 0.01 µg/mL and 1 µg/mL of eleutheroside B showed an increase in neurites, indicating that eleutheroside B induces the activation of neurites.

In addition, as shown in FIG. 11, as a result of measuring the neuronal differentiation ability of eleutheroside B in the neuroblast cell line, among the neuronal differentiation markers, sox2 and nestine representing the expansion phase, neuroD representing the differentiation phase, and neuN representing the integration phase showed a significant increase compared to the control, and DCX and Tuj1, which represent the differentiation, showed a slight increase compared to the control. From this, it was found that administration of eleutheroside B induces the activity for differentiation from neural stem cells into neural cells.

On the other hand, Example 8 of the present invention confirms the *in-vivo* efficacy, and shows remarkable improvement in cognitive function and the inhibition of astrocyte and microglia activity in Alzheimer's disease animal models.

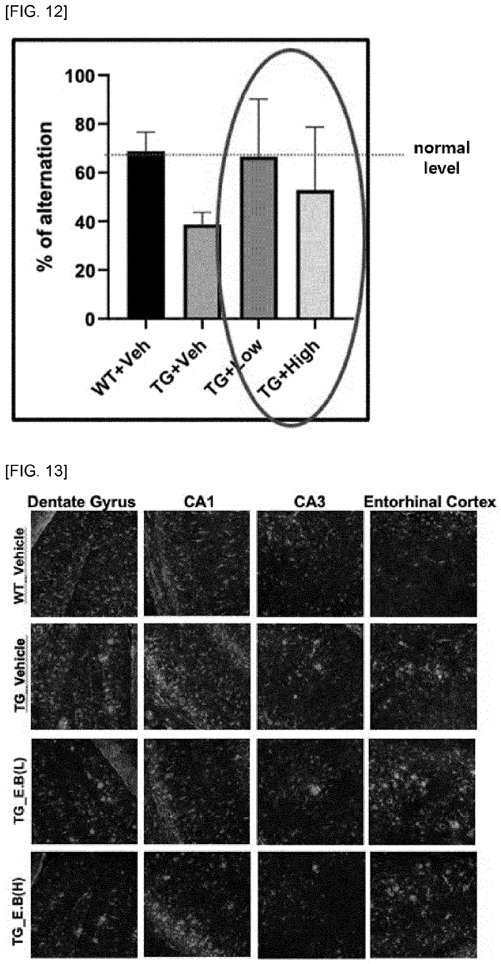

FIG. 12 shows the results of measuring working memory ability, which measures cognitive function, with Y-maze, and showed almost the same cognitive ability improvement as the control group in the group treated with eleutheroside B in the Alzheimer's disease animal model. As can be seen in FIG. 13 to FIG. 16, these results reflect the control of neuroinflammation by inhibiting the activity of astrocytes and microglia.

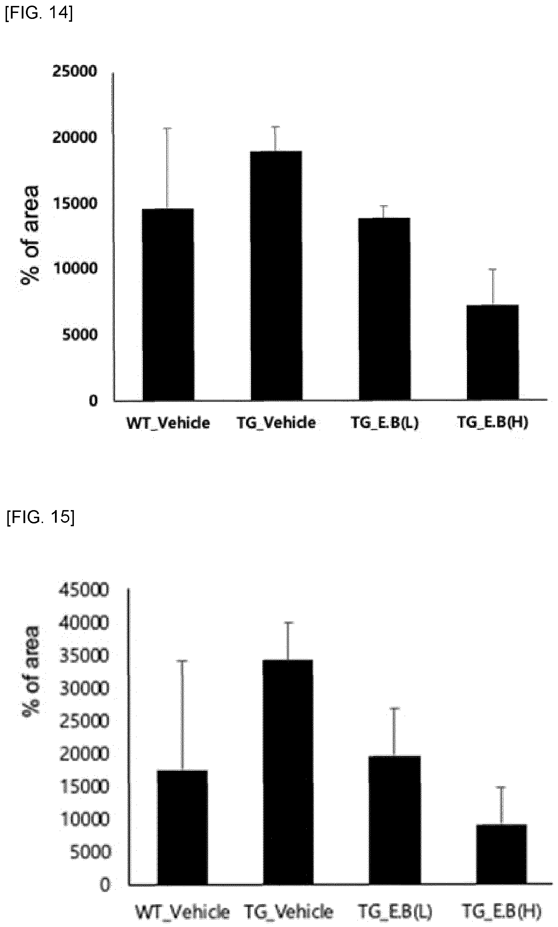

Specifically, FIG. 13 and FIG. 14 show the results of measuring the activity of astrocytes in 4 areas of the hippocampus, which are the main damaged areas in Alzheimer's disease, and quantifying the activity as the area of astrocytes, and they show that the activity of astrocytes is inhibited similarly or lower than the normal state in the group administered with eleutheroside B. FIG. 15 is a graph quantifying the degree of change in the activity of astrocytes in the CA1 region of the hippocampus by administration of eleutheroside B, which shows that inflammation in the CA1 region of the hippocampus, which is most important for cognitive ability, is greatly reduced.

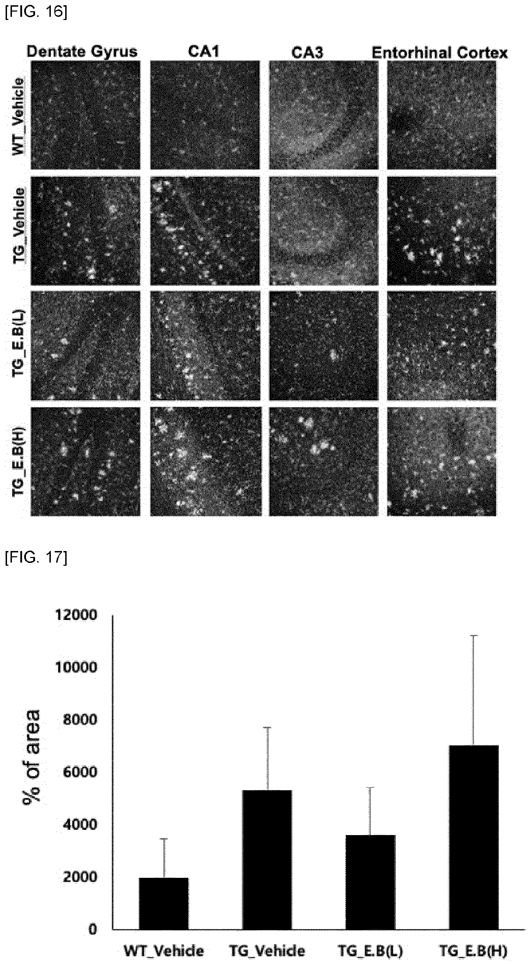

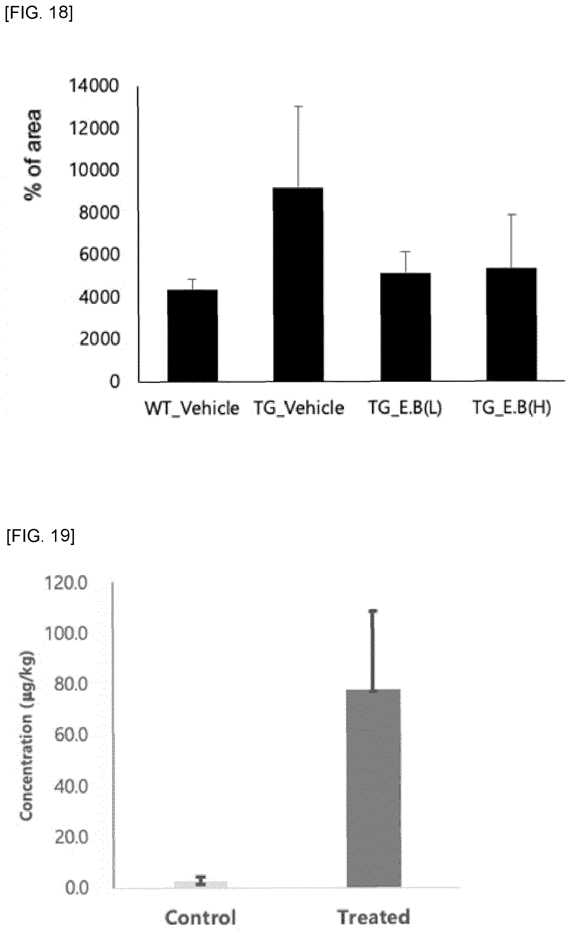

FIG. 16 and FIG. 17 show the results of measuring the activity of microglia in 4 areas of the hippocampus, which are the main damaged areas in Alzheimer's disease, and quantifying the activity as the area of microglia, and they show that the activity of microglia is suppressed more than the Alzheimer's disease animal model in the group administered with eleutheroside B. FIG. 18 is a graph quantifying the change of microglia in the CA1 region of the hippocampus by the treatment of eleutheroside B, which shows that the inflammation in the CA1 region of the hippocampus, which is very important for cognitive ability, is greatly reduced to almost the same as the normal state, shows, similar to astrocytes.

In addition, FIG. 19 is a quantitative result of LC-MS/MS that eleutheroside B passes through the BBB (blood brain barrier), which means that about 80 ppb of the drug is absorbed into the brain within 30 minutes, which means that eleutheroside B passes through the blood brain barrier. This is strong evidence that eleutheroside B is absorbed into the brain of genetically engineered mice for Alzheimer's disease to suppress the inflammation by inhibiting activities of astrocytes and microglia, resulting in cognitive improvement.

On the other hand, suitable carriers, excipients, and diluents commonly used for preparing pharmaceutical compositions may be further included. In addition, it may be formulated and used in the form of oral formulations such as powders, granules, tablets, capsules, suspensions, emulsions, syrups, aerosols, external preparations, suppositories, and sterile injection solutions according to conventional methods.

Carriers, excipients, and diluents that may be included in the composition include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil and the like. When formulating the composition, it may be prepared by using diluents or excipients such as commonly used fillers, extenders, binders, wetting agents, disintegrants, and surfactants.

The pharmaceutical composition according to the present invention can be administered in a pharmaceutically effective amount. In the present invention, "pharmaceutically effective amount" means an amount sufficient to treat a disease with a reasonable benefit/risk ratio applicable to medical treatment, and the effective dose level can be determined according to the type and severity of the patient's disease, the activity of the drug, the sensitivity to the drug, the time of administration, the route of administration and the rate of excretion, the duration of treatment, factors including drugs used concurrently, and other factors well known in the medical field.

In order to enhance the therapeutic effect, the pharmaceutical composition according to the present invention may be administered simultaneously, separately, or sequentially with drugs used in combination, and may be administered single or multiple times. Considering all of the above factors, it is important to administer an amount that can obtain the maximum effect with the minimum amount without side effects, which can be easily determined by those skilled in the art. Specifically, the effective amount of the pharmaceutical composition according to the present invention may vary depending on the patient's age, sex, condition, weight, absorption rate, inactivity rate and excretion rate of the active ingredient in the body, disease type, and concomitant drugs.

The pharmaceutical composition of the present invention can be administered to a subject by various routes. Administration can be carried out in any conceivable manner, for example, oral administration, intranasal administration, transbronchial administration, intraarterial injection, intravenous injection, subcutaneous injection, intramuscular injection, or intraperitoneal injection. The daily dose is preferably divided into one to several times a day, but it is not limited thereto.

The pharmaceutical composition of the present invention is determined according to the type of drug as an active ingredient, together with various factors such as the disease to be treated, the route of administration, the patient's age, sex, weight, and severity of the disease.

As another aspect of the present invention, the present invention provides a method for inhibiting neuroinflammation comprising administering the pharmaceutical composition to a subject. In the present invention, "subject" means a subject in need of treatment of a disease, and more specifically, means a mammal such as a human or non-human primate, mouse, dog, cat, horse, and cow.

### <Experimental example>

Hereinafter, the present invention will be described in more detail through examples. These examples are only intended to illustrate the present invention in more detail, and it will be apparent to those skilled in the art that the scope of the present invention is not limited by these examples according to the gist of the present invention. The examples of the present invention are provided to more completely explain the present invention to those of ordinary skill in the art.

### Example 1: Culture of astrocytes

The brains of 1-2 day old rats were excised, separated from the cerebral cortex, and cultured. That is, the mouse cortex was divided into single cells in high-glucose DMEM medium containing 10% FBS and penicillin-streptomycin solution (5000 units/ml penicillin, 5 mg/ml streptomycin, Corning, USA), and cultured in a 75-cm2T flask. (0.5 hemispheres/flask) in a cell incubator at 5% CO₂ and 37°C.

After 1 week of culture, microglia were removed by shaking at 250 rpm for 4 hours, and the cultured astrocytes were harvested using 0.1% trypsin to obtain an astrocyte population. Astrocytes were cultured in 6-well plates previously coated with Poly-D-Lysine (Sigma).

### Example 2: Microglia culture

BV2 cells were cultured in high glucose DMEM medium containing 10% FBS and penicillin-streptomycin solution (5000 units/ml penicillin, 5 mg/ml streptomycin, Corning, USA) in a cell incubator at 37°C in 5% CO₂, which was used by subculturing three times a week therein.

Cultured BV2 cells were harvested using 0.1 % trypsin to obtain a population, and cultured in 96-well plates pre-coated with Poly-D-Lysine (Sigma).

### Example 3: In-vitro brain disease cell culture

B6;SJL-Tg(APPSWE)2576Kha, a genetically modified mouse for Huntington's disease, was used as an *in-vitro* brain disease model. Neural stem cells were isolated from subventricular zone (SVZ) tissues of transgenic newborn mice and neurospheres were cultured. In addition, neurosphere cells were induced to attach to the bottom with 5% FBS+F12/DMEM medium for 48 hours, and to observe differentiation potential, eleutheroside B in 5% BS+F12/DMEM medium was added to the cells, and neurite activity was observed under a microscope. In addition, the neuronal cell differentiation potential using the Neuro-2a cell line, which is a neuroblast, was confirmed. To confirm differentiation, neural differentiation markers such as DCX, Tuj1, neuroD, and NeuN were confirmed by qPCR.

### Example 4: Confirmation of cytotoxicity of eleutheroside B to microglia

In order to check the cytotoxicity of eleutheroside B, BV2 cells (3×10⁵ cells/well) were cultured in a 96-well culture plate for 24 hours, and then treated with various concentrations of eleutheroside B, followed by incubation for 24 hours. After 24 hours, the medium was removed and WST-8 [2-(2-methoxy-4-nitrophenyl)-3-(4-nitrophenyl)-5-(2,4-disulfophenyl)-2H-tetrazolium (Cell Counting Kit-8 (CCK-8), dojindo)] was added at a concentration of 100uL/mL to induce a reduction reaction while incubating for 1 hour, and then absorbance (450 nm) was measured. Cell viability (%) (viability (%) = (treated group - negative control) / (positive control - negative control) × 100) was calculated using the eleutheroside B treated group and the untreated control group. According to the report that eleutheroside B showed cytotoxicity at 40 uM, the survival rate was confirmed up to 150 nM by diluting 1/2 from 40 uM.

As a result, the survival rate was 92±13.3% at 20 uM and 88±24.8% (mean±standard deviation) at 40 uM, and the survival rate tended to decrease. Therefore, it was confirmed that the safety was 100 times higher than the effective concentration (FIG. 1).

### Example 5: Verification of inhibitory effect on astrocytes activity according to eleutheroside B treatment

Astrocytes were used after primary culture was isolated from the brain of pup within 1 to 3 days after birth, and the separated astrocytes were treated with LPS at expressing the lowest concentration of IL-6. The drug treatment was performed simultaneously with LPS, and the activity of astrocytes was evaluated through the number of proliferated cells and changes in morphology (FIG. 2).

In addition, representative GFAP fluorescence intensities before and after drug treatment after induction of neuroinflammation through activation of astrocytes with LPS in astrocytes are shown in FIG. 3.

As shown in FIG. 3, after inducing neuroinflammation through activation of astrocytes with LPS in astrocytes, and treatment with 0.01 µg of eleutheroside B, the activation was reduced to almost the same intensity as the control, confirming that the inflammation was suppressed. In order to quantify the regulation of the inflammatory response by inhibiting the activation of astrocytes, high content imaging was analyzed by classifying into the number of proliferated cells, cell area, and GFAP fluorescence intensity, and the results are shown in FIG. 4.

As shown in FIG. 4, the activation of astrocytes by LPS and treatment of eleutheroside B at 1-10 nM drug concentration showed cell proliferation compared to LPS activity, and cell area and intensity of GFAP has been reduced. However, in the case of 1-10 nM drug concentration of donepezil, which is currently used as an Alzheimer's disease drug, compared to LPS activity, about 10% cell death was observed, cell area was increased, and almost the same or slight increase in GFAP intensity was observed. This shows that inflammation induced by the activation of astrocytes is not controlled by donepezil, which is currently used as a drug for Alzheimer's disease, but it can be alleviated by eleutheroside B.

In order to more accurately study the above effect, the secretion of cytokines was observed simultaneously with the effect of inhibiting the activity of astrocytes. After 24 hours of treatment with LPS and drugs, conditioned media were collected and changes in pro-inflammatory cytokines (TNFα) and anti-inflammatory cytokines (IL-10) were examined.

In order to simultaneously observe the secretion of these cytokines, a multiplexing method was used and measured in pg/mL units, and the results are shown in FIG. 5.

As shown in the multiplexing results of FIG. 5, an increase in cytokines by LPS was observed in the control, and the anti-inflammatory cytokine IL-10 was increased, but the pro-inflammatory cytokine TNF-α secretion was found to be decreased.

Therefore, it was found that eleutheroside B increases the secretion of anti-inflammatory cytokines and suppresses the secretion of pro-inflammatory cytokines to control inflammation, while donepezil was found to have no effect.

### Example 6: Verification of inhibitory effect on microglia activity according to eleutheroside B treatment

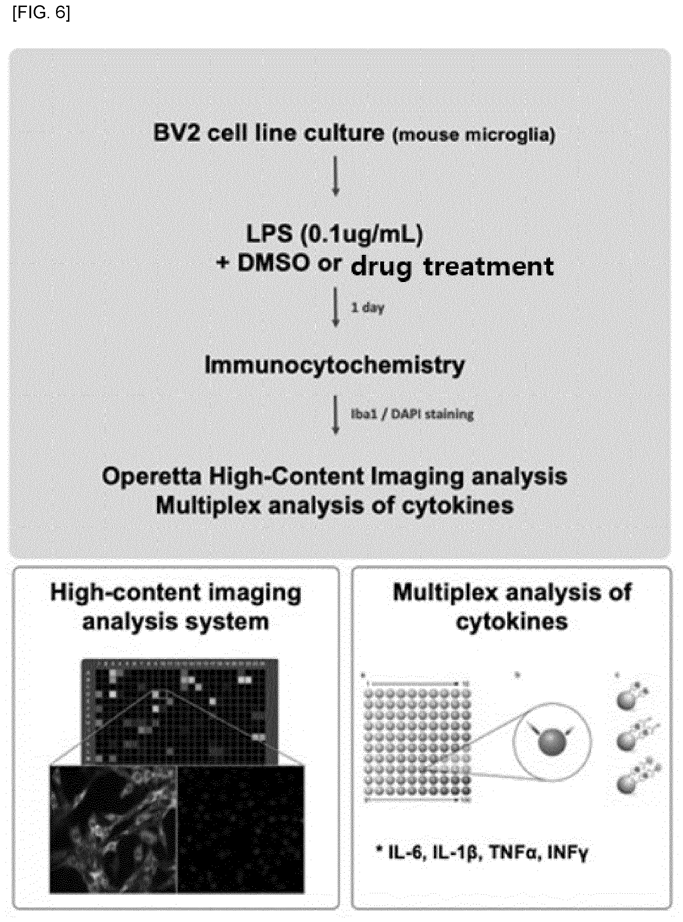

BV2 cells, a microglia cell line, were treated with LPS to induce microglia activation and inflammation, and eleutheroside B was treated at a concentration of 1 mM to 0.1 nM. The proliferation number and morphology changes after 24 hours of eleutheroside B treatment were quantified, and the activity level of microglial cells was verified by the fluorescence intensity of Iba1 (FIG. 6).

In addition, after the induction of neuroinflammation through the activation of microglial cells with LPS, representative Iba1 fluorescence intensities before and after drug treatment are shown in FIG. 7. As shown in FIG. 7, after inducing the activity of microglia with LPS and then treating with 10 nM of eleutheroside B, the activity was reduced to almost the same intensity as that of the control, confirming that inflammation was suppressed.

In order to confirm the regulation of the inflammatory response through the inhibition of microglia activity by eleutheroside B in a more precise way, high content imaging was analyzed by classifying into the number of proliferated cells and the fluorescence intensity of Iba1, and the results are shown in FIG. 8.

As shown in FIG. 8, after treatment of microglia with LPS, the number of cells decreased and the fluorescence intensity of Iba1 increased, showing a microglia activation pattern. However, after treatment with eleutheroside B, the number of cells increased, and a decrease in fluorescence intensity of Iba1 was observed.

This is a strong evidence that eleutheroside B can regulate microglia activity, and it was found that inflammation can be alleviated through the regulation of microglia activity by eleutheroside B.

In order to more accurately demonstrate the effect, the secretion of cytokines was simultaneously observed along with the effect of inhibiting the activity of microglial cells. To verify changes in cytokines, conditioned media were collected 24 hours after LPS and drug treatment on microglia, and changes in pro-inflammatory cytokine (TNFα) and anti-inflammatory cytokine (IL-10) were examined together, and the results are shown in FIG. 9.

As shown in FIG. 9, microglial cells were activated with LPS, treated with eleutheroside B and donepezil, and then multiplexed using conditioned media, and as a result, an increase in the anti-inflammatory cytokine IL-10 was confirmed, but a significant decrease in the pro-inflammatory cytokine TNF-α was not confirmed.

Therefore, it was found that eleutheroside B regulates inflammation through the increase of anti-inflammatory cytokines in microglia, but donepezil does not affect cytokine secretion.

### Example 7: Confirmation of neurite activity from neural stem cells according to eleutheroside B treatment

In order to confirm the activity of neurite from neural stem cells according to eleutheroside B treatment, B6;SJL-Tg(APPSWE)2576Kha, a genetically modified mouse for degenerative brain disease, was used as an *in-vitro* brain disease model. Neural stem cells were isolated from the subventricular zone (SVZ) tissue of genetically modified newborn mice, and the number of cells to be used in the experiment was secured through neurosphere culture.

Neurosphere cells were induced to adhere to the bottom with 5% FBS+F12/DMEM medium for 48 hours, and neurite activity was observed by treating cells with eleutheroside B in 5% BS+F12/DMEM medium for observation of differentiation potential, and the results are shown in FIG. 10.

As shown in FIG. 10, compared to the control group where almost no neurites were seen, the groups treated with 0.01 µg/mL and 1 µg/mL of eleutheroside B showed an increase in neurites and thus it was found that eleutheroside B induces the activation of the neurite.

In order to examine this at a molecular level, RNA analysis of neuronal differentiation markers (Sox2, Nestin, NeuroD, DCX, Tuj-1, NeuN) was performed, and the results are shown in FIG. 11.

When confirming the neuronal differentiation marker, a neuroblast cell line, Neuro-2a was used, and the differentiation was confirmed by qPCR to observe if the amoeba-type metaphase cells were differentiated into neurons by the drug. As shown in FIG. 11, among the neural differentiation markers, sox2 and nestine, which represent the expansion phase, neuroD, which represents the differentiation phase, and neuN, which represents the integration phase, showed significant increases compared to the control, and DCX and Tuj1, which represent differentiation, showed a slight increase compared to the control.

From these results, it was found that the administration of eleutheroside B promotes the neurite activity and the nerve cell differentiation.

### Example 8: Confirmation of in-vivo efficacy according to eleutheroside B treatment

Based on the *in-vitro* results of Examples 4 to 7, the *in-vivo* efficacy was confirmed using an Alzheimer's disease animal model mouse.

In 5XFAD, an animal model of Alzheimer's disease, amyloid protein is deposited from 2.5 months and cognitive function is impaired from 6 months. Accordingly, eleutheroside B (vehicle, 50 or 150 mg/kg) was intraperitoneally administered twice a week from 4 months, and working memory ability was measured at 6 months when cognitive function was impaired. The experimental groups were 3 animals each of WT+vehicle, TG+vehicle, TG+50mg/kg (TG_E.B(L)), and TG+150mg/kg (TG_E.B(H)) of the same age as TG.

Working memory ability was measured with Y-maze, and pre-adaptation training for the maze was performed the day before the maze experiment. Working memory performance was performed by sequentially moving through three passages for 8 minutes in a Y-shaped maze to check whether the movement to the previous passage was remembered, and the degree of cognitive impairment was confirmed by calculating the ratio of sequentially crossing the three passages.

As shown in FIG. 12, the group treated with eleutheroside B in the Alzheimer's disease animal model showed almost the same remarkable cognitive ability improvement as the control group.

In order to confime the pathological changes related to cognitive improvement, blood was removed by perfusion through the heart. The blood-free brain was fixed in 4% paraformaldehyde for one day, then made into a paraffin block and fabricated into 4 µm tissue sections for histological staining. The activities of astrocytes (GFAP) and microglia (Iba 1) were measured by confocal microscopy in the dentate gyrus, CA1, and CA3 regions of the hippocampus and the entorhinal cortex, which are major damaged areas in Alzheimer's disease.

As a result, as shown in FIG. 13, it was observed that the fluorescence intensity of GFAP, which indicates the degree of activity of astrocytes, showed a pattern similar to or lower than that of the normal state in the group administered with eleutheroside B. In order to quantify it, the area of activity was digitized using the MetaMorph program, and the average value is shown in FIG. 14 and FIG. 15. As shown in FIG. 14, in the group administered with eleutheroside B, the activity of astrocytes in the entire area of the hippocampus was surprisingly similar to or lower than that of normal cells in proportion to the dose, showing that inflammation was suppressed, and in particular, it was observed that the inflammation of CA1 of the hippocampus, which is most important for cognitive ability, was greatly reduced (FIG. 15).

In addition, as shown in FIG. 16, a pattern in which the fluorescence intensity of Iba 1, which indicates the degree of microglia activity, was lower than that of the Alzheimer's disease animal model was observed in the group administered with eleutheroside B. As shown in FIG. 17 quantifying this, the inflammation in the group administered with eleutheroside B was suppressed by inhibiting the activity of microglial cells in all areas of the hippocampus compared to the Alzheimer's disease animal model. Among them, it was observed that the inflammation of CA1 of the hippocampus, which is very important for cognitive ability, like astrocytes, was greatly reduced almost as in the normal state (FIG. 18).

### Example 9: Confirmation of passage of eleutheroside B through blood brain barrier (BBB)

The amount of eleutheroside B in the brain was measured using mass spectrometry to determine whether it could pass through the BBB. B6SJL, a wildtype of 5XFAD, was used as 3 mice each. It was administered intraperitoneally at 300 mg, and the brain of the rat was separated 30 minutes after administration. Eleutheroside B was extracted from the separated brain with 1 mL of ethanol using a homogenizer, and concentrated through speedvac. Impurities were removed from the concentrated sample using Sep-pak cartridge C18, and eleutheroside B was eluted with 1 mL of 80% ethanol. The eluted sample was concentrated using speedvac and analyzed using LC-MS/MS. The column used for HPLC was Agilent's Zorbox SB-C18 (1.8 µm × 2.1 mm) 5 cm, and the separation was performed at 0.4mL/min using a gradient program (5-90% B, 20min) using 0.1% ammonium hydroxide (in water, A) and acetonitrile (B) as the mobile phase. A triple quadrupole mass spectrometer was used, and quantification was performed using 371/209 (molecular ion/daughter ion) in negative MRM mode. For the calibration curve, the absolute calibration curve method was used. The measured concentrations are shown in FIG. 19. As shown in FIG. 19, the control was detected at almost several ppb, whereas the rats intraperitoneally administered with eleutheroside B had an average of 80 ppb, and it was confirmed that eleutheroside B efficiently passed the BBB within 30 minutes. Therefore, in connection with Example 8 above, eleutheroside B passes through the BBB to alleviate inflammation by regulating the activity of astrocytes and microglia, and in particular, it was found that by suppressing inflammation in the CA1 region of the hippocampus, which is very important for cognitive improvement, the improvement of cognitive ability was almost similar to that of the normal state.

All together, these results indicate that the pharmaceutical composition comprising eleutheroside B as an active ingredient according to the present invention passes through the BBB after administration and effectively inhibits the activity of active microglia and active astrocytes in the brain, and suppresses inflammation by increasing the secretion of anti-inflammatory cytokines or reducing pro-inflammatory cytokines, thereby protecting damage to brain nerve cells, and activates neurites in neural stem cells and promotes neural cell differentiation from neural stem cells and it was found that it has the effect of preventing or treating neurodegenerative diseases.

From the above description, those skilled in the art to which the present invention pertains will be able to understand that the present invention may be embodied in other specific forms without changing the technical spirit or essential characteristics thereof. In this regard, it should be understood that the embodiments described above are illustrative in all respects and not limiting. The scope of the present invention should be construed as including all changes or modifications derived from the meaning and scope of the claims to be described later and equivalent concepts rather than the detailed description above are included in the scope of the present invention.

## Claims

1. A pharmaceutical composition for treating neuroinflammatory disease in patients with increased activity of astrocytes and microglia, comprising eleutheroside B or a pharmaceutically acceptable salt thereof as an active ingredient.

2. The pharmaceutical composition of claim 1, wherein the composition activates neurites in neural stem cells and promotes differentiation of brain neurons.

3. The pharmaceutical composition of claim 1, wherein the neuroinflammatory disease is selected from the group consisting of Alzheimer's disease, Parkinson's disease, multiple sclerosis, cerebral infarction, stroke, epilepsy, multiple nerve atrophy, and Huntington's disease.

4. The pharmaceutical composition of claim 1, wherein the composition enhances a secretion of anti-inflammatory cytokines and inhibits a secretion of pro-inflammatory cytokines in astrocytes or microglia.

5. The pharmaceutical composition of claim 1, wherein the composition inhibits neuroinflammation of CA1 region of hippocampus induced by activity of microglia or astrocytes.
